(19) Europäisches Patentamt
European Patent Office
Office européen des brevets

(11) Publication number: **0 244 130 B1**

(12) # EUROPEAN PATENT SPECIFICATION

(45) Date of publication of the patent specification: **17.01.90**

(51) Int. Cl.⁴: **C 07 D 213/26**, C 07 D 213/30, C 07 D 213/32, C 07 D 213/06, C 07 D 213/08, C 07 D 405/04

(21) Application number: **87303372.4**

(22) Date of filing: **16.04.87**

(54) **Process for making 6-aryl-2-methyl pyridines.**

(30) Priority: **25.04.86 US 856601**

(43) Date of publication of application: **04.11.87 Bulletin 87/45**

(45) Publication of the grant of the patent: **17.01.90 Bulletin 90/03**

(84) Designated Contracting States: **AT BE CH DE ES FR GB IT LI NL SE**

(56) References cited:
**CH-A-647 508**

**CHEMICAL ABSTRACTS, vol. 103, no. 8, 19th August-2nd September 1985, page 565, abstract no. 53935k, Columbus, Ohio, US; V.G. KHARCHENKO et al.: "Reaction of alpha,alpha'-dichloro-substituted 1,5-diketones with ammonia and ammonium acetate", & KHIM. GETEROTSIKL. SOEDIN. 1985, (3), 352-354**

**CHEMICAL ABSTRACTS, vol. 97, no. 8, 8th-22nd November 1982, page 698, abstract no. 162783n, Columbus, Ohio, US; V.G. KHARCHENKO et al.: "Formation of nitrogenous heterocycles in the hydroamination of 1,5-diketones", & KHIM. GETEROSIKL. SOEDIN. 1982, (7), 944-947**

**J. HETEROCYCLIC CHEM., vol. 14, no. 147, 1977, pages 147-148; A. MARKOVAC et al.: "4-(3-Nitrophenyl)-2-picoline"**

(73) Proprietor: **ROHM AND HAAS COMPANY
Independence Mall West
Philadelphia Pennsylvania 19105 (US)**

(72) Inventor: **Barton, Alan Edward
2504 Oxford Court
Hatfield Pennsylvania 19440 (US)**

(74) Representative: **Angell, David Whilton
ROHM AND HAAS (UK) LTD. European Operations Patent Department Lennig House 2 Mason's Avenue
Croydon CR9 3NB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

EP 0 244 130 B1

LIBERGRAF, STOCKHOLM 1990

**Description**

This invention is concerned with a process for the preparation of 6-aryl-2-methylpyridines, at times referred to herein as 6-aryl-2-picolines.

The preparation of pyridines by reductive amination of 1, 5 diketone is disclosed in Chemical Abstracts *103* (1985) No. 53935k and 97 (1982) No.162783n.

The preparation of 6-aryl-2-picolines is generally reported in the literature to be accomplished by the coupling of diazotized haloanilines with 2-picoline, Chen et al., *Heterocycles*, 5, 239 (1976); Chen et al., *Bull. Inst. Chem. Acad. Sin.*, 25, 125 (1978); the coupling of nitrophenyldiazonium hydrochloride with 2-picoline, Agarawal et al., *J. of Med. Chem.*, 17, 631 (1974); and coupling of 2-picoline with cyclohexanone in the presence of an alkyllithium reagent followed by dehydrogenation, Bonnier et al., *Bull. Soc. Chim. Fr.*, No. 4, 1204 (1967).

These routes produce a low yield of particular isomers and require extensive chromatography to isolate substantially pure isomers. Further, the literature routes generally restrict the nature of the aryl group which is coupled to 2-picoline.

We have now found a process for making more isomerically homogenous 6-aryl-2-picolines in good overall yield. Further, the invention permits a broader choice of substituents on the aryl group.

According to this invention, a process for making 6-aryl-2-picolines having the formula

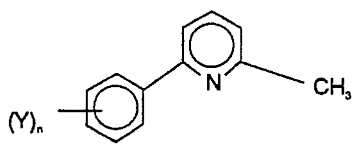

I

where Y is chloro, bromo, iodo, fluoro, nitro, $(C_1 - C_4)$alkyl, halo$(C_1 - C_4)$alkyl, $(C_1 - C_4)$alkoxy or $(C_1 - C_4)$alkylthio, or when two adjacent positions on the phenyl ring are substituted with alkoxy groups, these alkoxy groups may be joined to form, together with the carbon atoms to which they are both connected, a dioxolano or dioxano heterocyclic ring; and n is 0, 1, 2 or 3;

which comprises

(1) subjecting a ketal of 1-hydroxy-1-aryl-5hexanone to a Jones oxidation to yield a 1-aryl-1,5-hexanedione; or treating a ketal of 1-hydroxy-1-aryl-5hexanone with manganese dioxide in solvent to yield a ketone; and

(2) reacting the 1-aryl-1,5-hexanedione or the ketone from (1) with excess hydroxylamine hydrochloride in polar solvent at a temperature of from 50°C to 100°C to yield the 6-aryl-2-methylpyridine of Formula 1.

The ketal of 1-hydroxy-1-aryl-5-hexanone may be made by

(a) converting a 5-halo-2-pentanone derivative to the corresponding Grignard reagent or organolithium compound at a temperature of below about 100°C; and

(b) reacting the Grignard reagent or organolithium compound with a suitably substituted benzaldehyde at a temperature of from about -50°C to about 50°C in solvent.

The 6-aryl-2-methylpyridines made by this process are useful in preparing biologically active compounds.

In the invention one or more of the following preferred process parameters may be adopted: (i) the temperature in (a) is below 70°C; (ii) the temperature in (b) is -5 to 10°C; (iii) the solvent in (b) is ethereal; (iv) the Jones oxidation involves alcohol from (b), plus $CrO_3$ in water containing sulfuric acid; (v) the solvent in (1) comprises halogenated hydrocarbon; (vi) the temperature in (2) is 70 to 80°C; (vii) in step (b) the substituted benzaldehyde is added to the reaction product of step (a).

Examples of the ethereal solvent include diethyl ether and tetrahydrofuran (THF). Examples of the halogenated hydrocarbon solvent in (1) include dichloromethane, dichloroethane, chloroform and chlorobenzene, preferably dichloromethane. Examples of the polar solvent in (2) include acetonitrile, methanol, ethanol and dimethylformamide, preferably acetonitrile.

Schematically a preferred process of the present invention can be depicted as follows.

**Step a:**

Convert a 5-halo 2-pentanone derivative having the formula

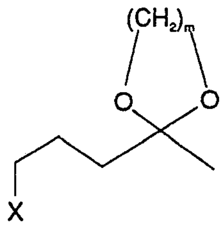

II

where X is halo (chloro, bromo or iodo) and m is 2 or 3; to the corresponding Grignard reagent or organolithium compound having the Formulae IIa and IIb respectively. Examples of the compounds of Formula II include 5-chloro-2-pentanone dimethyl ketal; 5-chloro2-pentanone ethylene ketal; 5-chloro-2-pentanone propylene ketal; 5-bromo-2-pentanone dimethyl ketal; 5-bromo-2-pentanone ethylene ketal; 5-bromo-2-pentanone propylene ketal; 5-iodo-2-pentanone dimethyl ketal; 5-iodo-2-pentanone ethylene ketal; 5-iodo-2-pentanone propylene ketal.

**Step b:**

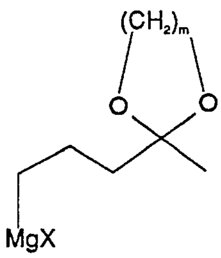

IIa

or

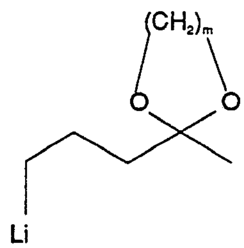

IIb

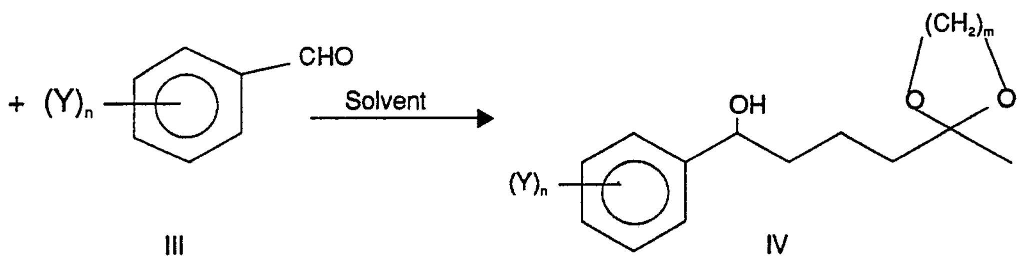


III

IV

**Step c:**

**Methode 1**

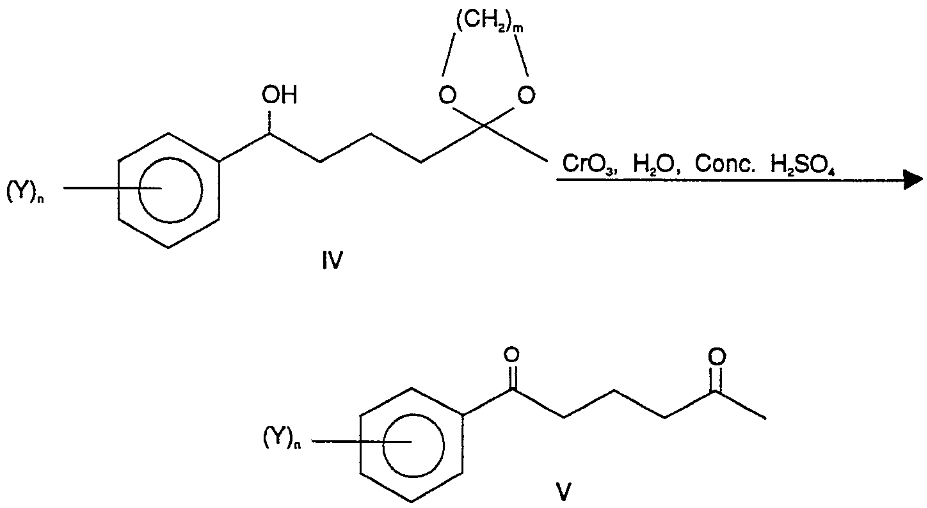


IV

V

**Method 2**

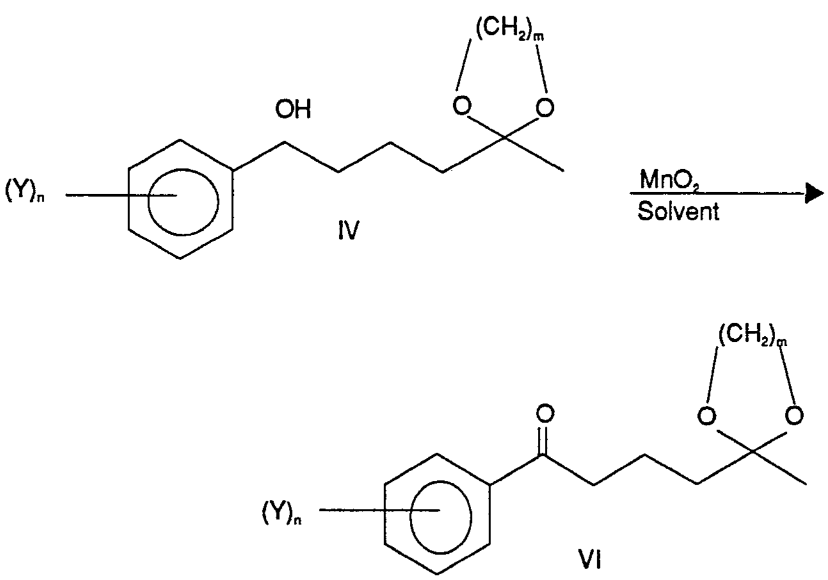


IV

VI

**Step d:**

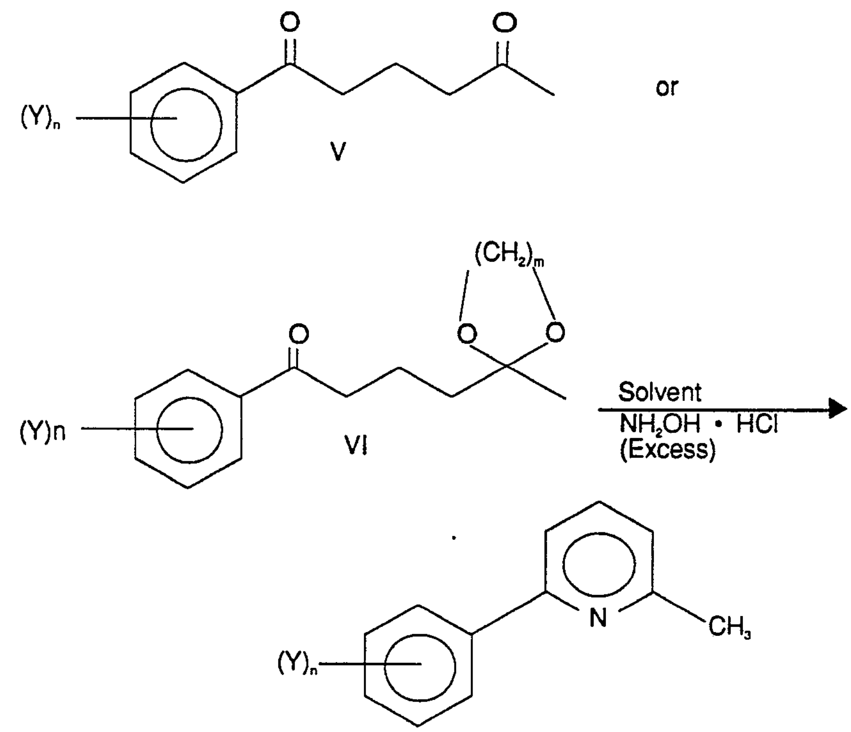


V

or

VI

I

where X is halo (chloro, bromo or iodo); m is 2 or 3; and Y and n are as defined above for Formula I.

In Step a, a 5-halo-2-pentanone derivative of Formula II is converted to the corresponding Grignard reagent of Formula IIa or organolithium compound of Formula IIb by a process as above described. Procedures for preparing the Grignard reagent or organolithium compound useful in Step a are known by those skilled in the art.

In Step b, the Grignard reagent or organolithium compound from Step 1 is reacted with a suitably substituted benzaldehyde of Formula III by a process as above described to yield the alcohol of Formula IV (ketal of 1-hydroxy-1-aryl-5-hexanone e. g. 1-hydroxy-1-aryl-5-hexanone-dialkylketal, 1-hydroxy-1-aryl-5-dioxolano-hexane or 1-hydroxy-1-aryl-5-dioxano-hexane).

In Step c, Method 1, the alcohol of Formula IV is conveniently subjected to the Jones oxidation by adding to the alcohol of Formula IV, $CrO_3$ in water plus concentrated sulfuric acid to yield the 1-aryl-1,5-hexanedione of Formula V.

In Step c, Method 2, an alternative especially for use when chromium reagents cannot be used, for example, where Y is an alkylthio group such as methylthio, the alcohol of Formula IV is treated with manganese dioxide in a suitable solvent as above described.

In Step d, the 1-aryl-1,5-hexanedione of Formula V or the ketone of Formula VI is treated with excess hydroxylamine hydrochloride by a method as above described, to yield the 6-aryl-2-methylpyridine of Formula I. By "excess" hydroxylamine hydrochloride in Step (d) is meant generally from about 1.5 equivalents to about 5 equivalents, preferably from about 1.5 equivalents to about 2 equivalents of hydroxylamine hydrochloride per equivalent of the 1-aryl-1,5-hexanedione of Formula V or the ketone of Formula VI.

The compounds of Formula III and the 5-halo-2-pentanone derivatives are commercially available or can be prepared by known procedures.

Substantially equimolar amounts of reactants are preferably used in Steps a to d, except in Step d where an excess of hydroxylamine hydrochloride is employed, although higher or lower amounts can be used if desired.

Modifications to the above process may be necessary to accommodate reactive functionalities of particular materials. Such modifications would be readily apparent to those skilled in the art.

Preferably, the process of the invention is carried out at about atmospheric pressure, although higher or lower pressures can be used if desired.

The 6-aryl-2-methylpyridines prepared by the process of the present invention can be isolated or further reacted to prepare biologically active compounds such as certain of the 6-aryl-pyridine thiosemicarbazones disclosed in the literature references cited above and certain of the insecticidally active compounds disclosed in our copending application entitled Insecticidal 6-arylpyridine thiosemicarbazones filed on the same day as the present application and published as EP-A-0 243 101. Preparation of said biologically active thiosemi-carbazones from 6-aryl-2-methylpyridines by general synthesis routes are either known or described in the above-cited references.

The following examples illustrate this invention. The structure of the compounds afforded by the process of the Examples was confirmed by NMR and in some cases by IR and/or elemental analysis and was either an oil or a low-melting solid. Stated yields are based on the amount of benzaldehyde used.

**Example 1 - Preparation of 6-(3-bromophenyl)-2-methyl pyridine**

5-chloro-2-pentanone ethylene ketal (0.0665 mol, 10 ml) was added to a suspension of magnesium (0.075 mol, 1.8 g, cleaned with ethylene dibromide) in 10 ml tetrahydrofuran (THF) so as to maintain the internal reaction temperature below 60°C. After the addition was complete, the green-grey reaction mixture was heated to about 55°C for a further 2 hours. After cooling to about 5°C and addition of a further 10 ml THF, a solution of 3-bromobenzaldehyde (0.05 mol, 9.2 g) in 10 ml THF was added so as to maintain the internal temperature below 10°C. After warming the mixture to room temperature, it was poured onto aqueous saturated ammonium chloride. Extraction with ether followed by drying of the extracts and evaporation of solvents afforded 18 g of a pale yellow oil.

The crude ketal-alcohol was dissolved in 200 ml of acetone, cooled to about 5°C and treated with Jones reagent (30 ml). After the addition was complete, the heterogenous mixture was allowed to stand at room temperature for 1.5 hours. The acetone layer was decanted and the residue was extracted with ether. The combined organic extracts were washed with several portions of dilute ammonium hydroxide and then were dried. Evaporation of the solvents afforded 15 g of crude 1-aryl-1,5-hexanedione. The crude diketone was dissolved in 120 ml of acetonitrile and treated with hydroxylamine hydrochloride (0.06 mol, 4.2 g) and heated at 70 - 80°C for 16 hours. After cooling to room temperature, the mixture was partitioned between saturated aqueous sodium bicarbonate and ether. The aqueous layer was extracted with ether and the combined extracts were washed with water and dried. Evaporation of solvents yielded 6.5 g of crude pyridine which was chromatographed on silica gel using 20 % ether-hexanes as eluant to give 6.0 g of 6-(3-bromophenyl)-2-methyl pyridine (49 %).

**Example 2 - Preparation of 6-phenyl-2-methylpyridine**

The procedures of Example 1 were substantially followed except benzaldehyde was used to afford 6-phenyl-2-methylpyridine (40 %).

**Example 3 - Preparation of 6-(2-tolyl)-2-methylpyridine**

The procedures of Example 1 were substantially followed except 2-methylbenzaldehyde was used to afford 6-(2-tolyl)-2-methylpyridine (31 %).

**Example 4 - Preparation of 6-(2-trifluoromethylphenyl)-2-methylpyridine**

The procedures of Example 1 were substantially followed except 2-trifluoromethylbenzaldehyde was used to afford 6-(2-trifluoromethylphenyl)-2-methylpyridine.

**Example 5 - Preparation of 6-(2-methoxyphenyl)-2-methylpyridine**

The procedures of Example 1 were substantially followed except 2-methoxybenzaldehyde was used to afford 6-(2-methoxyphenyl)-2-methylpyridine (30 %).

**Example 6 - Preparation of 6-(3-nitrophenyl)-2-methyl-pyridine**

The procedures of Example 1 were substantially followed except 3-nitrobenzaldehyde was used to afford 6-(3-nitrophenyl)-2-methylpyridine.

**Example 7 - Preparation of 6-(4-bromophenyl)-2-methyl-pyridine**

The procedures of Example 1 were substantially followed except 4-bromobenzaldehyde was used to afford 6-(4-bromophenyl)-2-methylpyridine.

**Example 8 - Preparation of 6-(2-fluorophenyl)-2-methyl-pyridine**

The procedures of Example 1 were substantially followed except 2-fluorobenzaldehyde was used to afford 6-(2-fluorophenyl)-2-methylpyridine.

**Example 9 - Preparation of 6-(2-chlorophenyl)-2-methyl-pyridine**

The procedures of Example 1 were substantially followed except 2-chlorobenzaldehyde was used to afford 6-(2-chlorophenyl)-2-methylpyridine.

**Example 10 - Preparation of 6-(4-methylthiophenyl)-2-methylpyridine**

The crude alcohol was prepared substantially following the procedures of Example 1 for preparing said alcohol except 4-methylthiobenzaldehyde was used.

The crude alcohol from addition of Grignard reagent to *p*-methylthiobenzaldehyde (7.2 g) was dissolved in 250 ml $CH_2Cl_2$. 70 g of manganese dioxide was added and the heterogeneous reaction mixture was stirred at 23°C for about 50 hours. The entire reaction mixture was filtered through Celite and the filtrate was evaporated to give 6.3 g of ketone-ketal.

Crude ketone-ketal (4.2 g) was dissolved in approximately 50 ml acetonitrile and treated with hydroxylamine hydrochloride (2.0 g). The mixture was heated at 70 - 80°C for 6 hours. The entire reaction mixture was poured into a saturated solution of sodium bicarbonate and extracted twice with 100 ml ether. The organic extracts were washed with brine, treated with charcoal and dried over magnesium sulfate. Evaporation afforded a red/brown oil which was chromatographed on silica gel using 35 % ether hexanes as eluant to give 1.55 g of 6-(4-methylthiophenyl)-2-methylpyridine as a yellow oil (50 %).

**Example 11 - Preparation of 6-(2,4-dichlorophenyl)-2-methylpyridine**

The procedures of Example 1 were substantially followed except 2,4-dichlorobenzaldehyde was used to afford 6-(2, 4-dichlorophenyl)-2-methylpyridine.

**Example 12 - Preparation of 6-(2-chloro-4,5-dioxolano-phenyl)-2-methylpyridine**

The procedures of Example 1 were substantially followed except 2-chloro-4,5-dioxolanobenzaldehyde was used to afford 6-(2-chloro-4,5-dioxolanophenyl)-2-methylpyridine.

**Claims:**

1. A process for making a 6-aryl-2-methylpyridine of the formula

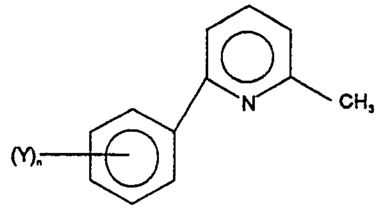

(I)

where Y is chloro, bromo, iodo, fluoro, nitro, ($C_1$ - $C_4$)alkyl, halo($C_1$ - $C_4$)alkyl, ($C_1$ - $C_4$)alkoxy or ($C_1$ - $C_4$)alkylthio, or when two adjacent positions on the phenyl ring are substituted with alkoxy groups, these alkoxy groups may be joined to form, together with the carbon atoms to which both are attached a dioxolano or dioxano heterocyclic ring; and n is 0, 1, 2 or 3;

which comprises

(1) subjecting a ketal of 1-hydroxy-1-aryl-5hexanone to a Jones oxidation to yield a 1-aryl-1,5-hexanedione; or treating the 1-aryl-1-hydroxy-5-ketalhexane with manganese dioxide in solvent to yield a ketone; and
(2) reacting the 1-aryl-1,5-hexanedione or the ketone from (a) with excess hydroxylamine hydrochloride in polar solvent at a temperature of from 50°C to 100°C to yield a 6-aryl-2-methylpyridine.

2. The process of claim 1 wherein the ketal of 1-hydroxy-1-aryl-5-hexanone is a 1-aryl-1-hydroxy-5dioxanohexane.

3. The process of claim 2 wherein the 1-aryl-1-hydroxy-5-dioxano-hexane comprises 1-aryl-1-hydroxy-5-cyclopentylidene-ketal-hexane or 1-aryl-1-hydroxy-5-cyclohexylidene-ketal-hexane.

4. The process of any preceding claim wherein the ketal of 1-hydroxy-1-aryl-hexanone is prepared by converting a 5-halo-2-pentanone derivative to the corresponding Grignard reagent or organolithium compound at a temperature of below 100°C; and adding a suitably substituted benzaldehyde at a temperature of from -50°C to 50°C in solvent.

5. The process of any preceding claim wherein the solvent in (1) comprises halogenated hydrocarbon.

6. The process of claim 4 or 5 wherein the 5-halo-2-pentanone derivative is converted to the corresponding Grignard reagent at a temperature below 70°C.

7. The process of claim 6 wherein the benzaldehyde is added at a temperature of from -5°C to 10°C.

8. The process of any preceding claim wherein (2) is carried out at a temperature of from 70°C to 80°C.

9. The process of any preceding claim wherein from 1.5 equivalents to 5 equivalents of hydroxylamine hydrochloride is used per equivalent of the 1-aryl-1,5-hexanedione or the ketone from (1).

10. The process of claim 9 wherein from 1.5 equivalents to 2 equivalents of hydroxylamine hydrochloride is used.

**Patentansprüche**

1. Verfahren zur Herstellung eines 6-Aryl-2-methylpyridins der Formel

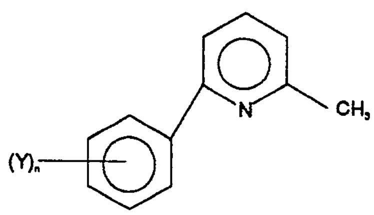

(I)

worin Y Chlor, Brom, Jod, Fluor, Nitro, ($C_1$-$C_4$)Alkyl, Halogen-($C_1$-$C_4$)-alkyl, ($C_1$-$C_4$)-Alkoxy oder ($C_1$-$C_4$)-Alkylthio ist, oder dann, wenn zwei benachbarte Positionen an dem Phenylring mit Alkoxygruppen substituiert sind, diese Alkoxygruppen verbunden sein können um zusammen mit den Kohlenstoffatomen, mit welchen sie jeweils verknüpft sind, einen Dioxolano- oder Dioxano-Heterozyklus zu bilden, und n 0, 1, 2 oder 3 ist,
welches darin besteht,

(1) eine Ketal von 1-Hydroxy-1-aryl-5-hexanon einer Jones-Oxidation zur Gewinnung eines 1-Aryl-1,5-hexandions zu unterziehen oder das 1-Aryl-1-Hydroxy-5-ketalhexan mit Mangandioxid in einem Lösungsmittel zur Gewinnung eines Ketons zu behandeln und
(2) das 1-Aryl-1,5-Hexandion oder das Keton aus (a) mit einem Überschuß Hydroylaminhydrochlorid in einem polaren Lösungsmittel bei einer Temperatur von 50°C bis 100°C zur Gewinnung eines 6-Aryl-2-methylpyridins umzusetzen.

2. Verfahren nach Anspruch 1, wobei das Ketal des 1-Hydroxy-1-aryl-hexanons ein 1-Aryl-1-hydroxy-5-dioxanohexan ist.

3. Verfahren nach Anspruch 2, wobei das 1-Aryl-1-hydroxy-5-dioxanohexan aus 1-Aryl-1-hydroxy-5-cyclopentylidenketalhexan oder 1-Aryl-1-hydroxy-5-cyclohexylidenketalhexan besteht.

4. Verfahren nach einem der vorhergehenden Ansprüche, wobei das Ketal von 1-Hydroxy-1-arylhexanon hergestellt wird durch Umwandlung eines 5-Halogen-2-pentanonderivats in das entsprechende Grignard-Reagens oder Organolithiumverbindung bei einer Temperatur unterhalb 100°C und ein in geeigneter Weise substituierter Benzaldehyd bei einer Temperatur von -50°C bis 50°C in einem Lösungsmittel zugesetzt wird.

5. Verfahren nach einem der vorhergehenden Ansprüche, wobei das Lösungsmittel in (1) aus einem halogenierten Kohlenwasserstoff besteht.

6. Verfahren nach Anspruch 4 oder 5, wobei das 5-Halogen-2-pentanonderivat in das entsprechende Grignard-Reagens bei einer Temperatur unterhalb 70°C umgewandelt wird.

7. Verfahren nach Anspruch 6, wobei der Benzaldehyd bei einer Temperatur von -5°C bis 10°C zugesetzt wird.

8. Verfahren nach einem der vorhergehenden Ansprüche, wobei (2) bei einer Temperatur von 70°C bis 80°C durchgeführt wird.

9. Verfahren nach einem der vorhergehenden Ansprüche, wobei 1,5 Äquivlante bis 5 Äquivalente Hydroxylaminhydrochlorid pro Äquivalent 1-Aryl-1,5-hexandion oder Keton aus (1) verwendet werden.

10. Verfahren nach Anspruch 9, wobei 1,5 Äquivalente bis 2 Äquivalente Hydroxylaminohydrochlorid verwendet werden.

**Revendications**

1. Un procédé pour préparer une 6-aryl-2-méthylpyridine de formule

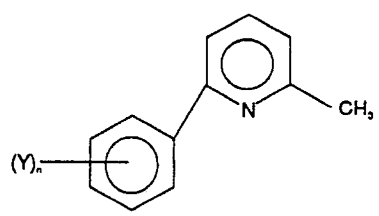

(I)

dans laquelle Y est un chloro, bromo, iodo, fluoro, nitro, alcoyle en $C_1$-$C_4$, halogénoalcoyle en $C_1$-$C_4$, alcoxy en $C_1$-$C_4$ ou alcoylthio en $C_1$-$C_4$ ou, lorsque deux positions adjacentes sur le cycle phényle sont substituées par des groupes alcoxy, ces groupes alcoxy peuvent être réunis pour former, avec les atomes de carbone auxquels ils sont tous deux fixés, un hétérocycle dioxolanno ou dioxanno; et n vaut 0, 1, 2 ou 3;
qui consiste à

(1) soumettre un acétal de 1-hydroxy-1-aryl-5-hexanone à une oxydation de Jones pour former une 1-aryl-1,5-hexanedione; ou traiter le 1-aryl-1-hydroxy-5-acétalhexane avec du dioxyde de manganèse dans un solvant pour produire une cétone; et
(2) faire réagir la 1-aryl-1,5-hexanedione ou la cétone de (a) avec un excès de chlorhydrate d'hydroxylamine dans un solvant polaire à une température de 50°C à 100°C pour produire une 6-aryl-2-méthylpyridine.

2. Le procédé de la revendication 1, dans lequel l'acétal de 1-hydroxy-1-aryl-5-hexanone est un 1-aryl-1-hydroxy-5-dioxannohexane.

3. Le procédé de la revendication 2, dans lequel le 1-aryl-1-hydroxy-5-dioxannohexane comprend un 1-aryl-1-hydroxy-5-cyclopentylidène-acétalhexane ou un 1-aryl-1-hydroxy-5-cyclohexylidèneacétalhexane.

4. Le procédé de l'une quelconque des revendications précédentes, dans lequel l'acétal de la 1-hydroxy-1-arylhexanone est préparé par conversion d'un dérivé de 5-halogéno-2-pentanone en le réactif de Grignard ou un composé organolithien correspondants, à une température inférieure à 100°C; et addition d'un benzaldéhyde convenablement

substitué à une température de -50°C à 50°C dans un solvant.

5. Le procédé de l'une quelconque des revendications précédentes, dans lequel le solvant de (1) comprend un hydrocarbure halogéné.

6. Le procédé de la revendication 4 ou 5, dans lequel le dérivé de 5-halogéno-2-pentanone est transformé en le réactif de Grignard correspondant à une température inférieure à 70°C.

7. Le procédé de la revendication 6, dans lequel le benzaldéhyde est ajouté à une température de -5°C à 10°C.

8. Le procédé de l'une quelconque des revendications précédentes, dans lequel (2) est effectué à une température de 70°C à 80°C.

9. Le procédé de l'une quelconque des revendications précédentes, dans lequel on utilise de 1,5 à 5 équivalents de chlorhydrate d'hydroxylamine par équivalent de la 1-aryl-1,5-hexanedione ou de la cétone de (1).

10. Le procédé de la revendication 9, dans lequel on utilise de 1,5 équivalent à 2 équivalents de chlorhydrate d'hydroxylamine.